# EUROPEAN PATENT SPECIFICATION

(11) **EP 1 436 057 B1**
(45) Date of publication and mention of the grant of the patent: **19.04.2006**
(21) Application number: 02801781.2
(22) Date of filing: 17.10.2002
(51) Int. Cl.: B01D 9/00

(54) **ROTOR-STATOR APPARATUS AND PROCESS FOR THE FORMATION OF PARTICLES**
ROTOR-STATOR APPARAT UND VERFAHREN ZUR BILDUNG VON PARTIKELN
APPAREIL A ROTOR-STATOR ET PROCEDE DE FORMATION DE PARTICULES

(30) Priority: 17.10.2001 US 329641 P
(43) Date of publication of application: 14.07.2004
(73) Proprietor: E.I. DU PONT DE NEMOURS AND COMPANY, Wilmington, DE 19898 (US)
(72) Inventor: CALABRESE, Richard, V., Laurel, MD 20723 (US); DALZIEL, Sean, Mark, Wilmington, DE 19809 (US); GOMMEREN, Erik, Henricus, Jacobus, Cornelis, Hockessin, DE 19707 (US); FRIEDMANNN, Thomas, Hockessin, DE 19707 (US)
(74) Representative: Hughes, Andrea Michelle
(86) International application number: PCT/US2002/033307
(87) International publication number: WO 2003/033097

(56) References cited:
- EP-A- 0 514 890
- EP-A- 1 065 301
- WO-A-01/14036
- US-A- 3 529 936
- US-A- 4 187 143

## Description

### FIELD OF THE INVENTION

This invention relates to the use of a high intensity, in-line rotor-stator apparatus to produce fine particles via precipitation or crystallization.

### BACKGROUND OF THE INVENTION

The production of fine particles is used in many applications, such as oral, transdermal, injected or inhaled pharmaceuticals, biopharmaceuticals, nutraceuticals, diagnostic agents, agrochemicals, pigments, food ingredients, food formulations, beverages, fine chemicals, cosmetics, electronic materials, inorganic minerals and metals. Only a few current precipitation and crystallization techniques work reliably to produce fine crystals having a narrow size distribution, and often milling, crushing, or grinding are required, as a post treatment, to reduce the crystallized particles to the desired size and distribution ranges.

Milling, grinding, and crushing, however, impose limitations including contamination of the product by grinding tools, degradation of heat sensitive materials during grinding, lack of brittleness of some solids (e.g., most polymers, proteins, polysaccharides, etc), chemical degradation due to exposure to the atmosphere, long processing times and high-energy consumption.

It would, therefore, be advantageous to prepare fine particles (around 10 µm or less), especially in the sub-micron and nano-size range, having consistent and controlled physical criteria, including particle size and shape, quality of the crystalline phase, chemical purity, and enhanced handling and fluidizing properties, without the need to further mill, grind or crush the product. In particular, the pharmaceutical field has a pronounced need for an apparatus and/or method capable of large-scale production of sub-micron and nano-sized particles.

In the pharmaceutical field, high bioavailability and short dissolution time are desirable, and often necessary, attributes of the end products produced. A large proportion of small molecule pharmaceuticals are poorly soluble in water or gastric fluids. Thus, to increase dissolution rate and bioavailability, they need to be reduced in particle size so as to increase the surface area. Conventional batch (or continuous) crystallization processes, if modified to enable a high supersaturation environment to generate fine sized crystals with high surface area, causes a broad size distribution and poor crystal formation. The conventional batch processes do not provide high quality crystals since such processes simply recirculate the solution in a tank, wherein the solution may or may not pass through the high-shear zone. Consequently, the products have low purity, high friability and decreased stability and inadequate bioavailability unless further treated. In order to produce an end product having increased purity and a more stable crystal structure, a slow crystallization technique has been utilized.

A slower crystallization process, however, decreases the productivity of the crystallization apparatus and produces large, low surface area particles that require subsequent high intensity milling. Currently, pharmaceutical compounds often require post-crystallization milling to increase particle surface area and therefore bioavailability. For the reasons stated above, however, post-crystallization milling is an undesirable step in producing fine particles. As a result, the large-scale production of end-products having high surface area, high chemical purity, and high stability without post-crystallization milling is not obtainable through current crystallization technology.

One crystallization process involves the use of impinging jet nozzles, whereby two jet nozzles are positioned so as to allow fluid jet streams that are discharged from each jet nozzle to intersect midway between the jet nozzles from which they are discharged. One of the fluid jet streams is comprised of a medicament dissolved in a solvent, while the other fluid jet stream is comprised of an anti-solvent. This crystallization process is designed to produce very fine particles (e.g., approximately 10 µm and less); however, it presents several difficulties and limitations in its utility.

First, the mixing energy inside an impinging jet apparatus is controlled by the velocity of the two impinging fluid streams. Such high velocities are only practically achievable at low production rates, where very fine bore jets are used. Since the linear velocity (1-dimension) of the fluid streams and their volumetric flow rate (3-dimensional) do not scale linearly with increasing jet diameter, scale-up of impinging jet apparatus is commonly unsuccessful above rates of several kilograms of product per hour. Therefore, impinging jet nozzles are only suitable to discharge very fine fluid streams at low production rates. Secondly, it is very difficult to align, and maintain the alignment of these impinging fluid jet streams. Again, if the diameter of the jets is increased to accommodate an increased production rate, the dissipation of energy during mixing is less controllable, making scale-up complicated or unsuccessful. Thirdly, various parts of the impinging jet apparatus used to produce the crystallized particles tend to clog easily with both crystallized, as well as, foreign materials. Finally, although the impinging jet crystallization process can be utilized to produce fine medicinal substances with particle sizes around 10 µm and less, such a process requires multiple units for larger scale production of fine particles making it a very costly approach to production. They require additional operators and increased complexity with regulatory requirements on batch records and lot documentation. Hence impinging jet crystallization/precipitation is not a practical alternative for the larger scale production of fine particles. (See, for example, WO 01/14036).

The present invention, however, provides an efficient, simple and easily scaled-up apparatus and process for producing fine particles, wherein a very high mixing intensity can be delivered and controlled over a very short residence time. One advantage of the present invention is that it enables higher volume processes to harness the advantages equivalent to intense mixing delivered by impinging jet systems. Another advantage is that it does not suffer the blockage and complicated alignment limitations of impinging jets.

Antisolvent crystallization/precipitation, otherwise referred to as drowning out or watering-out, is a widely discussed and industrially used process for causing a substance that has been dissolved in a liquid to precipitate out of the liquid. (See, for example, "Crystallization" by J.W. Mullin, 3^{rd} edition, Butterworth Hienemann 1992, or "Perry's Chemical Engineers' Handbook", edited by D. W. Green and J. O. Maloney, 6^{th} edition, McGraw-Hill Book Co., NY, 1984). The method involves the addition of a second liquid comprising an anti-solvent to a first liquid comprising a solvent and a substance dissolved in the solvent. The two liquids are miscible and lead to a lowering of solubility of the material to be crystallized in the mixed solvents. As a result, the substance dissolved in the first liquid crystallizes out of the liquid, and can subsequently be isolated if required.

Currently, rotor-stator mixers are occasionally used as a grinding device following a regular crystallization process. Additionally, rotor-stator mixers have been used, directly, or indirectly after a crystallization unit operation to disperse, attrit or change the shape of previously prepared crystals. Prior to the present invention, rotor-stator mixers had not been utilized as part of a single step crystallization/precipitation process that produces fine (<10 micron) or ultra-fine (sub-micron and nano-sized) particles that do not need to be ground in a further post-crystallization/precipitation grinding step.

Rotor-stator mixers are used in many industries, including the food industry. Food items such as mixed dairy products, mayonnaise, and the like can be produced with these devices.

Rotor-stator mixers are high-speed stirring devices wherein the rotor portion is a stirrer blade, and the stator portion is a container with openings through which materials pass into an outer housing and then out of the system. The stator is generally sized for close tolerance with the rotor portion. Alignment is not an issue with rotor-stator mixers since the manufacturing techniques to produce them is well established, and the inlet, outlet and stator openings allow for streams larger than those of impinging jets. However, currently available standard rotor-stator mixers provide only one inlet port for fluid streams entering the system.

US 4,187,143 and EP 1065301 disclose a rotor-stator apparatus where both the rotor and stator are ring-shaped.

The present invention provided herein is a rotor-stator apparatus that allows multiple fluid streams containing different fluids to be fed into the rotor-stator apparatus so that the different fluids do not intimately mix until inside the high shear zone of the mixer. This creates an environment whereby nucleation and crystal/precipitate growth occur over a controlled and very short time period. As a result, the crystals/precipitate produced according to the process and apparatus of the present invention are smaller in size and have a narrower size distribution range than could be obtained by mixing the two liquids in a conventional stirred tank type crystallizer.

Still another advantage of the present invention is that it may be optionally utilized to enable feeding of a stream containing seed crystals or other particles for co-precipitation, further growth or coating; as well as in the production of small, high surface area particles that can be used as carrier particles for liquids.

Thus; the invention provides a crystallization or precipitation process and apparatus that enables controlled formation of fine crystals/particles. Based on the particular parameters discussed herein, the apparatus and process according to the present invention are also able to control the size and shape of the crystals/particles formed during the crystallization/precipitation process. This invention further allows for in-line use, thereby enabling larger-scale productions of materials than has previously been available. It is believed that the use of an in-line rotor-stator mixer in a crystallization/precipitation process to achieve intense micromixing is novel.

Potential applications of this technology are very broad, for example, industries able to utilize the particles generated by the present invention include pharmaceuticals, nutraceuticals, diagnostics, agrochemicals, pigments, food ingredients, food formulations, beverages, chemicals, cosmetics, electronic materials, inorganic minerals and metals.

### SUMMARY OF THE INVENTION

Claimed herein is a rotor-stator apparatus as defined in claim 1, equipped with at least two inlet pipes capable of introducing at least two separate fluid streams (including solvents, liquids, slurries, suspensions and the like) to produce nano- to micron-size particles via crystallization/precipitation.

Also claimed is a process preferably utilizing the apparatus as described herein, for the crystallization/precipitation of a substance that is dissolved or suspended in a solvent, wherein the dissolved substance is caused to crystallize/precipitate out of said solution substantially simultaneously and substantially immediately on being mixed with the anti-solvent in a high shear zone.

The present apparatus and process allow for the direct and immediate production of acceptable nano and micron-sized crystals/precipitate that exhibit greatly reduced particle size, increased surface area, improved uniformity of shape, lack of roughened surfaces or surface charge (as often develops on milled materials) improved stability, purity and uniformity. The nano- and micron-sized crystals/precipitate produced also have a high surface area, enabling the crystals/precipitate produced to meet the bioavailability needs of the pharmaceutical industry without having to undergo a post-crystallization grinding step. The present invention, therefore, provides a quicker, less expensive, and more efficient way to produce acceptable nano- and micron-sized crystals/precipitate for several industry segments; including particularly the pharmaceutical area.

### BRIEF DESCRIPTION OF THE FIGURES

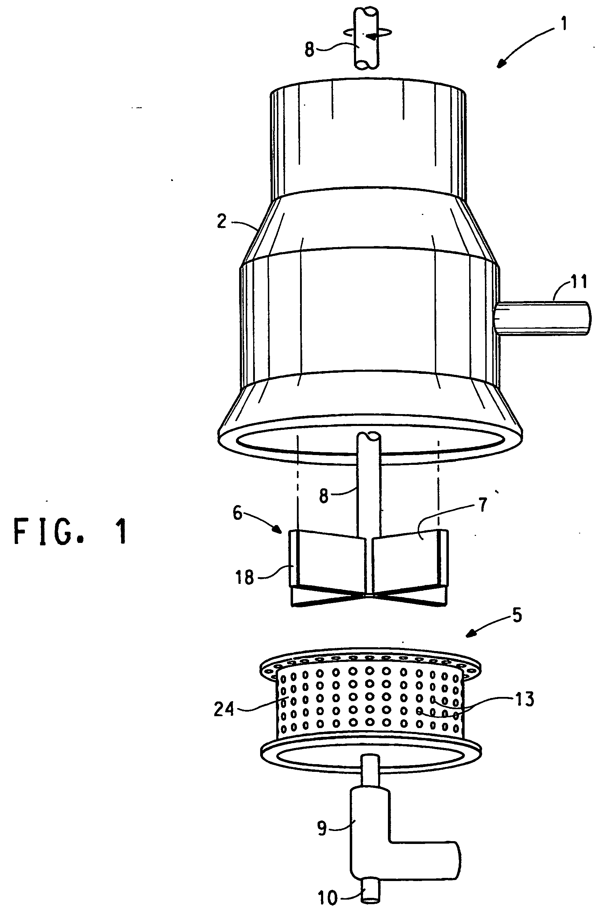

Figure 1 is an exploded view of an embodiment of the present invention.
Figure 1B is a longitudinal cross-section of an embodiment of the present invention showing a second configuration of the inlet pipes.

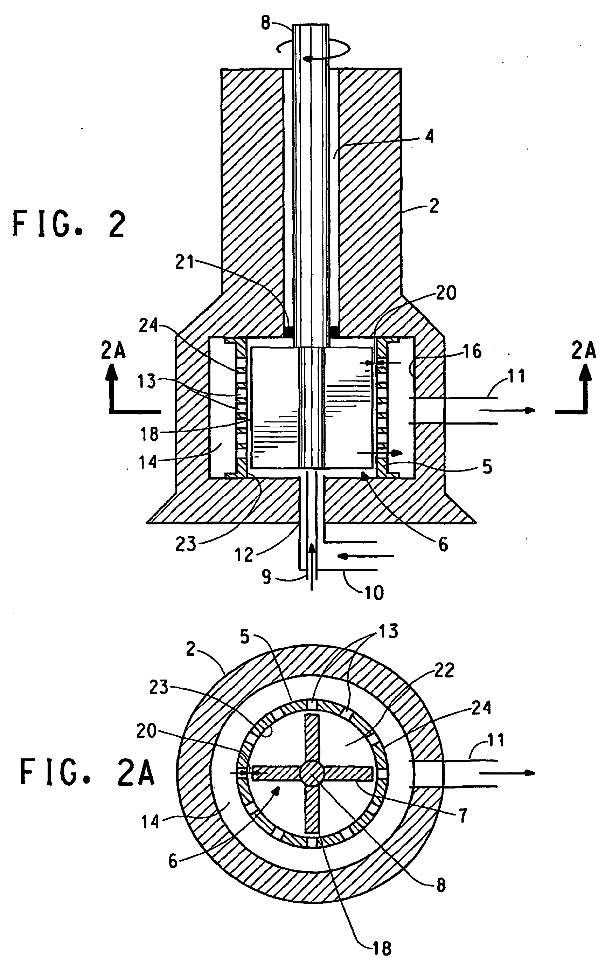

Figure 2 is a longitudinal cross-section of an embodiment of the present invention.
Figure 2A is a top cross-sectional view of the present invention.

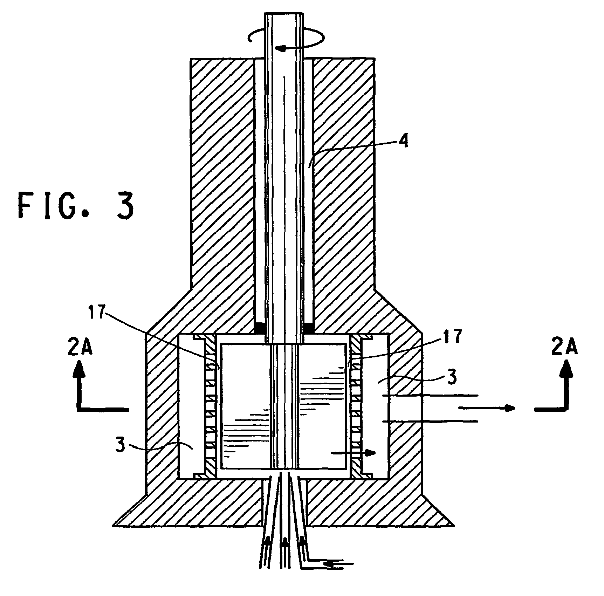

Figure 3 is a longitudinal cross-section of an embodiment of the present invention having angled inlet pipes.
Figure 4 is a diagram showing the recirculation embodiment of the present invention.

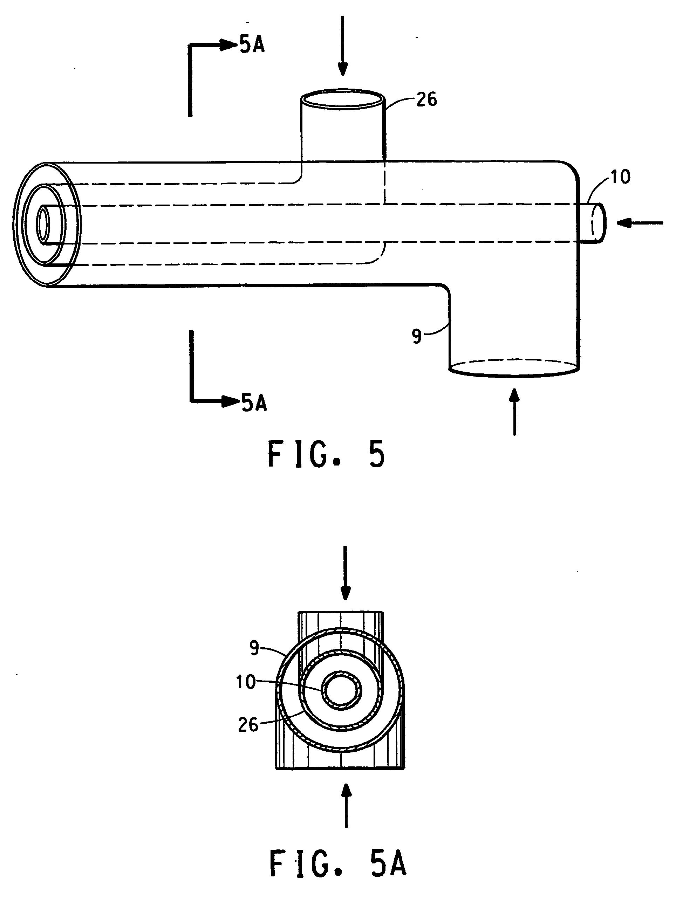

Figure 5 is a side view showing the multi-axial or nested inlet pipes.
Figure 5A is a top view of the multi-axial or nested inlet pipes.

### DETAILED DESCRIPTION OF THE INVENTION

The present invention is an in-line rotor-stator apparatus and a crystallization/precipitation process using said apparatus to obtain crystals/precipitate on a nanometer or micron scale. The material passes through the apparatus in a continuous fashion. The product is formed by mixing two or more fluid streams, at least a first and a second fluid, in a high-shear zone. After formation, the crystals/precipitate produced are collected. Generally, lab-scale mixers can produce up to about a liter of material per minute, with scale-up to larger volumes (e.g. scale-up factors up to at least one hundred times) capable of being accomplished.

Although the present invention may be utilized for the production of any precipitated or crystallized particles, including pharmaceuticals, biopharmaceuticals, nutraceuticals, diagnostic agents, agrochemicals, pigments, food ingredients, food formulations, beverages, fine chemicals, cosmetics, electronic materials, inorganic minerals and metals; for ease of description, principally pharmaceuticals will be specifically addressed. The crystalline/precipitated particles for other industry segments can be produced using the same general techniques described herein as easily modified by those skilled in the art.

As used herein, a "rotor-stator apparatus" includes the preferred embodiment disclosed herein, as well as the alternative embodiments, such as those utilizing multiple rotors, rotors having teeth colloidal mill rotors, multiple concentric stators, roughened surface, teeth or textured stators and the like.

As used herein, the term "high-shear zone" shall include all areas within the present invention that are subject to the high shear force adjacent to a stationary surface, including, that area between the at least one rotor blade tip and the interior portion of the stator wall (also known as the "shear gap"), the stator slots or apertures, the jets emanating from the stator into the volute and the rotor-swept volume.

As used herein, the term "shear force" shall encompass all of the mixing/dispersion, mechanical forces produced in the apparatus of the invention including, but not limited to, the nominal shear rate in the gap between the rotor and stator, elongation forces, turbulence, cavitation, and impingement of the stator slot surfaces.

As used herein, the terms "crystallization" and/or "precipitation" include any methodology of producing particles from fluids; including, but not limited to, classical solvent/antisolvent crystallization/precipitation; temperature dependent crystallization/precipitation; "salting out" crystallization/precipitation; pH dependent reactions; "cooling driven" crystallization/precipitation; crystallization/precipitation based upon chemical and/or physical reactions; etc.

As used herein, "biopharmaceutical" includes any therapeutic compound being derived from a biological source or chemically synthesized to be equivalent to a product from a biological source, for example, a protein, a peptide, a vaccine, a nucleic acid, an immunoglobulin, a polysaccharide, cell product, a plant extract, an animal extract, a recombinant protein an enzyme or combinations thereof.

As used herein "solvent" and "anti-solvent" denote, respectively, those fluids in which a substance is dissolved, and any fluid which causes the desired substance to crystallize/precipitate or fall out of solution. Accordingly the antisolvent can also mean the reactant fluid in reactive chemistry, the precipitate causing fluid in precipitation processes, the precipitate causing fluid in a salting out process, and the cooling liquid conditions in cooling driven processes.

A preferred embodiment of the apparatus (1) of the present invention comprises a housing (2) having within it a first cavity (3), and preferably, a second cavity (4). The first cavity (3) is able to accommodate a stator (5); a rotor (6) having at least one blade (7), wherein the rotor is connected to a rotatably mounted drive shaft (8); at least two inlet pipes (9 and 10); at least one entry port (12); and an outlet orifice (11).

The rotor-stator apparatus (1), and accordingly the housing (2) and the other various components, can be constructed of any generally non-reactive material having sufficient rigidity to withstand the pressures and forces created within the apparatus (1) during its use including, but not limited to, stainless steel. The size of the rotor-stator apparatus (1) is limited only by good engineering practices.

As previously noted, the housing has a first cavity (3) and a second cavity (4), wherein the first cavity (3) contains both the liquid and mechanical components of the present invention and wherein the second cavity (4) simply allows for passage of the drive shaft (8) through the housing (2) to be connected with a motor. The first and second cavities are separated from one another by a seal (21), which prevents any fluids contained in the first cavity (3) from being released into the second cavity (4).

The stator (5) of the preferred embodiment of the present invention surrounds the rotor (6) and comprises an outer wall portion (24) and an interior wall portion (23) and is typically stationary within the housing (2). The stator (5) may be of any shape, provided that the shape of the rotor (6) provides the requisite distance between the at least one rotor blade tip (18) and the inner wall portion (23) of the stator. Preferably, however, the shape of the stator (5) is cylindrical, and thus for ease of description this shape will be the only shape described herein; however, skilled artisans will recognize and understand that modifications to the rotor, at least one blade, drive shaft and stator must be made should another shape be utilized. The stator (5) must be of a size capable of accommodating a spinning rotor (6) within it, an internal volume known as the rotor-swept volume (22), however, the at least one rotor blade tip (18) and the interior wall portion of the stator (23) must be in very close proximity to one another to produce the necessary shear force required for intimate mixing to occur, a distance known as the "shear gap".

Alternatively, the present invention may utilize multiple stators, wherein said stators are concentric cylinders. The multiple cylinders are generally configured so one of the cylinders, preferably the inner cylinder, rotates while the outer cylinder is preferably stationary and has a roughened surface, profile and/or texture, modifications or teeth thereby causing an increase in the shear force when compared to the single cylindrical stator configuration. For ease of description, only the preferred stator embodiment will be specifically addressed. The shear gap width and the shear force will remain consistent with the disclosure provided herein and crystalline/precipitated particles can be produced using the same general techniques described herein as easily modified by those skilled in the art.

Furthermore, the stator (5) has numerable apertures (13), also known as slots, thereby allowing the passage of the at least two fluids through its wall. These apertures (13) may be of any shape and/or size including, but not limited to, slots, circular, triangular, or square or mixtures thereof. The apertures (13) are located at positions directly in-line with the at least one rotor blade (7). This ensures that the fluids pass through the high shear zone, thereby resulting in intimate mixing and short time frames over which nucleation will occur. The size and/or shape of the aperture (13) does not affect the size or shape of the crystals produced in accordance with the present invention, but are influential in the production of the shear force due to their affect on the flow pattern of the fluid within the apparatus, however the main parameters are the shear gap width and the blade tip speed. The size and shape of the crystals may be manipulated by changing the chemistry of the fluids streams, the rotor rpm, the flow rates of the various inlet streams and their flow rates relative to one another.

The rotor (6) of the present invention comprises, at least one blade. In addition, the present invention may have multiple rotors and/or stators wherein such an arrangement would further serve to increase the shear force acting within the apparatus; and such variations of the rotor-stator are included within the scope of the claimed invention. For ease of description, only the preferred rotor embodiment will be specifically addressed. The shear gap width and the shear force will remain consistent with the disclosure provided herein and crystalline/precipitated particles can be produced using the same general techniques described herein as easily modified by those skilled in the art. The rotor comprises at least one blade (7), which preferably extends radially. The rotor (6) is connected to a rotatably mounted drive shaft (8). The drive shaft (8), in turn, is generally connected to a motor or driving force capable of rotating the rotor (6) at speeds sufficient to cause crystallization/precipitation. The shape of the at least one rotor blade (7) is not critical for the present invention so long as the blade (7) is capable of providing the requisite blade tip speed along the height of the stator where the apertures are located and the at least one blade tip is the required distance from the interior wall portion (23) of the stator.

The revolutions per minute (RPM) of the rotor vary with the scale of the apparatus of the present invention. Generally, the maximum allowable RPM decreases as the apparatus increases in size. Thus, the shear forces of the present invention are more dependent upon blade tip speed rather than RPM's. Typically, the blade tip speed is up to about 50 meters per second, preferably between about 0.2 meters per second and about 50 meters per second, and generally remains in this range for apparatuses of differing sizes. For example, a 35mm apparatus may be run at about 10,000 RPM, while a 330mm apparatus may run at about 1,200 RPM, however these apparatuses will have substantially equivalent blade tip speeds, calculated using the formula: 2 x pi x RPM/60 x radius.

The rotatably mounted drive shaft (8) may be a solid shaft, or conversely, may be hollow to allow it to act as a single or multiple inlet pipe to deposit the at least two fluid streams within the rotor-swept volume (22). Similarly, the rotor (6) itself may also be hollow, wherein the at least two fluid streams may be fed through the rotor (6) and dispersed at several points along the rotor (6), for example, along the at least one rotor blade (7) and/or blade tip (18).

In particular, two aspects of the present invention are critical to generating the shear force necessary for good mixing and formation of fine, narrowly sized crystals/precipitates: the width of the shear gap (20), which is the distance between the at least one blade tip and the interior portion (23) of the stator wall, and the tip speed of the at least one blade tip. The width of the shear gap typically ranges between about 0.01 mm and about 10mm, depending upon the size of the apparatus being utilized, such that as the size of the apparatus increase, the shear gap width also increases. Preferably, however, the gap width of the present invention is about 1 mm. Generally, smaller shear gap widths (20) in conjunction with higher blade tip speeds result in finer crystals, however, the size and/or shape of the crystals/precipitate are affected by both the chemistry of the solution utilized as well as the fluid dynamics of the present invention. The blade tip speed is the circumferential speed with which the at least one blade tip rotates within the stator, wherein blade tip speed is generally up to about 50 meters per second, preferably between about 0.2 meters per second and about 50 meters per second. The nominal shear rate generated by the invention may range widely and is generally, up to about 1,000,000 reciprocal seconds and is dependent upon the solvent, anti-solvent and dissolved substance used in the process. However, skilled artisans will recognize and understand that the shear force may be varied in accordance with the manipulation of other variables.

The at least two inlet pipes (9 and 10) enter the rotor-stator apparatus (1) at the at least one entry port (12). The multiple inlet pipes may be of any diameter, as long as they are of a size to allow the necessary number of fluid streams to be deposited in the rotor-swept volume (22), while also accommodating the necessary flow rates. It is preferred, but not required, that the inlet pipes have equivalent cross-sectional areas. The number of inlet pipes is limited only by the space available on the unit.

The inlet pipes (9 and 10) provide at least two fluid streams, at least a first fluid having at least one substance dissolved with it and at least one second fluid, capable of producing crystals/precipitate when intimately mixed by the shear forces generated by the present invention. The at least two inlet pipes (9 and 10) may be utilized in numerous configurations including, for example, but not limited to, coaxial or nested inlet pipes having varying diameters (i.e. inlet pipe 1 is an inner pipe that is smaller than, and inserted through, inlet pipe 2, which is a larger outer pipe, or further, where inlet pipe 3 (26) may be axially aligned within inlet pipe 2 which is axially aligned within inlet pipe 1), adjacent inlet pipes, annularly positioned inlet pipes, and the like. It should be noted that when used in the coaxial configuration, while generally there is one inner pipe for each outer pipe, more than one inner pipe could be used, in either a manifold, or multi-axial fashion. The at least two inlet pipes should introduce the at least two fluids into the rotor-swept volume in close proximity to the rotor. However, the fluids are not mixed together prior to entering the high shear zone and the inlet pipes (9 and 10) deposit the fluids within the rotor-swept volume (22), which is critical for the production of very fine particles. Intimate mixing of the at least two fluid streams therefore occurs in the high-shear zone (17). Intimate mixing occurs at the smallest scales of turbulent motion; the more intense the mixing, the smaller the scales of turbulent motion.

The at least one entry port (12), and consequently the at least two inlet pipes, may be positioned anywhere on the housing (2), so long as the fluids are fed into the rotor-swept volume (22); for example, they may be positioned all on the same side of the housing, on opposite sides of the housing, on adjoining sides of the housing, or any combinations thereof. Moreover, the inlet pipes (9 and 10) may feed into the rotor-stator apparatus (1) at any angle so long as the fluids do not come into substantial contact with one another before entering the rotor-swept volume (22) and the high-shear zone (17). The apparatus (1) may have coaxial inlet pipes (9 and 10) as described above thereby allowing more than one inlet pipe, as well as, more than one fluid to enter through the same inlet pipe, or may only.have one inlet pipe, and therefore one fluid. Preferably, however, the inlet pipes (9 and 10) deposit the at least two fluid streams directly beneath and/or directly above the rotor (6).

Generally, the apparatus (1) operates by having at least two fluid streams travel into the apparatus via the at least one entry port (12) and through and the at least two inlet pipes (9 and10) to introduce the at least two fluid streams into the apparatus, unless such inlet pipes are multi-axial. The fluid streams are deposited inside the rotor-swept volume (22) and fed to the rotor (6). The fluids are caused to rapidly rotate within the stator (5) due to the high-speed rotation of the rotor (6). The centrifugal force that is generated by the spinning rotor, and aided by the shear gap, transports the fluids in a radial direction towards the wall of the stator and eventually through the apertures (13), also referred to as stator slots, in the stator wall (15) and into the volute (14), which is the annular gap between the outer wall portion (24) of the stator (15) and the inner wall (16) of the housing (2). As the fluids approach the apertures (13) in the stator wall (15), the fluids enter into a high-shear zone (17), wherein the shear force is generated by the high circumferential speed of the at least one rotor blade tip (18) and the shear gap width (19). At this point the fluids become intimately mixed due to the shear force and crystallization/precipitation occurs. The fluid streams are further mixed as the now single mixture is still subjected to the shear force as the mixture is forced through the stator wall apertures (13) and into the volute (14). Subsequent to passing through the apertures (13) in the stator wall (5), the newly formed crystals/precipitate are transported through the volute and towards the outlet orifice (11) for collection, further reaction or isolation.

In the precipitation/crystallization process of the present invention, the means of introducing the two fluids into the rotor-stator apparatus plays an important role in the dissipation of supersaturation. For conventional crystallizers/precipitators, as commonly used for high volume processes, when the resulting crystals/precipitates are desired to be large (e.g. a mean size of 50 µm or greater), a stirred tank crystallizer with antisolvent addition is typically used. This uses conventional equipment, for example when anti-solvent addition occurs through a dip-tube and is mixed by a low speed large agitator, such as a pitched blade, marine or hydrofoil type impeller. This typically gives a product of broad size distribution and larger crystals/precipitates. The reason for the larger crystal/precipitate size and the broad distribution is that there is continual internal recycling of all crystallizing particles through the mixing zone in a stirred tank type crystallizer. This leads to nucleation occurring for at least the total length of time that the anti-solvent stream is added (minutes to hours per batch) and subsequent recycle of nuclei results in their growth up to larger crystals and a broad distribution of sizes (over hours typically). This type of equipment can be considered using the stirred tank reactor model for mixing. In contrast, the present invention is a continuous steady-state flow-through process. Hence in this present invention, the nucleated crystals are denied the opportunity for further growth while the anti-solvent is being added (except for the several seconds of their residence time), since they continuously transfer through this process. It has been observed in practice that the rates of nucleation and crystal/precipitate growth are highly dependent upon the manner and timing of mixing of the fluid streams being co-fed through the inlet pipes into the rotor-stator unit. A high mixing intensity, i.e, high speed movement of the rotor blades, over a defined short period of time leads to more intimate mixing and higher nucleation rates. Higher nucleation rates cause formation of fine crystals/precipitates having a narrower size distribution than crystals/precipitates formed by lower nucleation rates. Until discovering the present invention, however, high nucleation rates over short time periods were only capable of being harnessed industrially on a small-scale (mostly in the pharmaceutical industry) by using the anti-solvent crystallization impinging jet process, as discussed above.

The present crystallization/precipitation process and apparatus enables high nucleation rates to be utilized in the large-scale production of fine crystals/precipitates without all of the problems associated with the impinging jet method or the detriments of attempting to generate fine crystals from conventional stirred tank type crystallizers.

The crystallization/precipitation process of the present invention allows the habit of the crystals to be controlled by manipulating the high shear zone. For example, common crystal habits include, but are not limited to, cubic, needle-like, plate-like, prismatic, and elongated prisms. The particular habit of a crystal is partly related to the relative supersaturation at the growing face. Intimate mixing of liquid streams leads to more uniform supersaturation distributions and correspondingly more uniform face growth rates on the crystals and a more uniform crystal habit. In addition, depending on the rotor-stator design and operation, there is the possibility of breakage of crystals in the rotor-stator mixer, which also leads to a differentiated crystal habit. In particular, such breakage will reduce most habits into an equant prism-like shape, less needle-like or plate-like. Both intimate mixing and breakage may be related to the effects observed.

The precipitation/crystallization process of the present invention also enables control of crystal size. The size range of crystals that may be formed by the process of the present invention is typically 100 nm to 100 µm. The preferred size of the crystals is 100 nm to 10 µm with a narrow distribution range. The size of the crystals that are produced in the process and apparatus according to this invention are related to the mechanical properties of the apparatus and its operational settings as well as the solubility, growth, nucleation and reaction properties of the chemical system being used.

Crystal habit and size formation is more clearly demonstrated by the examples set forth below. The habits and sizes demonstrated in the examples are specific to the example material under the tested conditions, and are not limitations to be placed on any other substances that may be crystallized or precipitated.

In the process of the present invention, the choice of solvent depends upon the solubility of the substance to be dissolved. Preferably, a substantially saturated or supersaturated solution is obtained upon the mixing of the fluid streams injected through their respective inlet pipes. As is consistent with antisolvent crystallization/precipitation techniques known to persons skilled in the art, at least one fluid is typically a solvent containing the substance to be precipitated, and the at least one associated second fluid is an antisolvent. In all cases, the antisolvent should be substantially miscible with the solvent in order to form a single liquid-phase solvent mixture, while the substance to be precipitated should be poorly soluble in the antisolvent so that upon contact, the dissolved substance is precipitated out of the first fluid.

The process and apparatus of the present invention can be utilized to crystallize a wide variety of pharmaceutical substances. The water soluble and water insoluble pharmaceutical substances that can be crystallized according to the present invention include, but are not limited to, anabolic steroids, analeptics, analgesics, anesthetics, antacids, anti-arrthymics, anti-asthmatics, antibiotics, anti-cariogenics, anticoagulants, anticolonergics, anticonvulsants, antidepressants, antidiabetics, antidiarrheals, anti-emetics, anti-epileptics, antifungals, antihelmintics, antihemorrhoidals, antihistamines, antihormones, antihypertensives, anti-hypotensives, anti-inflammatories, antimuscarinics, antimycotics, antineoplastics, anti-obesity drugs, antiplaque agents, antiprotozoals, antipsychotics, antiseptics, anti-spasmotics, anti-thrombics, antitussives, antivirals, anxiolytics, astringents, beta-adrenergic receptor blocking drugs, bile acids, breath fresheners, bronchospasmolytic drugs, bronchodilators, calcium channel blockers, cardiac glycosides, contraceptives, corticosteriods, decongestants, diagnostics, digestives, diuretics, dopaminergics, electrolytes, emetics, expectorants, haemostatic drugs, hormones, hormone replacement therapy drugs, hypnotics, hypoglycemic drugs, immunosuppressants, impotence drugs, laxatives, lipid regulators, mucolytics, muscle relaxants, non-steroidal anti-inflammatories, nutraceuticals, pain relievers, parasympathicolytics, parasympathicomimetics, prostagladins, psychostimulants, psychotropics, sedatives, sex steroids, spasmolytics, steroids, stimulants, sulfonamides, sympathicolytics, sympathicomimetics, sympathomimetics, thyreomimetics, thyreostatic drugs, vasodialators, vitamins, xanthines, and mixtures thereof.

As previously noted, the process and apparatus of the present invention can also be utilized to crystallize/precipitate a wide variety of other industrial substances, such as, for example foods and food ingredients. The water soluble and water insoluble foods and food ingredients that can be crystallized or precipitated include, but are not limited to, carbohydrates, polysaccharides, oligosaccharides, disaccharides, monosaccharides, proteins, peptides, amino acids, lipids, fatty acids, phytochemicals, vitamins, minerals, salts, food colors, enzymes, sweeteners, anti-caking agents, thickeners, emulsifiers, stabilizers, anti-microbial agents, antioxidants, and mixtures thereof.

When precipitating a soy protein, it is preferred to introduce into the high-shear zone an acid, such as hydrochloric acid or phosphoric acid, or an organic acid, such as, citric acid, malic acid as the antisolvent. Another preferred combination of fluids for precipitating soy protein involves introducing an acid beverage into the high-shear zone as the antisolvent, resulting in a finished product containing the precipitated protein.

When precipitating a milk protein, it is preferred to introduce an acid beverage into the high-shear zone as the antisolvent, resulting in a finished product containing the precipitated protein.

When precipitating a vitamin, mineral or other fortifying ingredient, it is preferred to introduce a food, food ingredient or beverage (pure or dissolved) into the high-shear zone as the precipitating agent, resulting in a finished product containing the precipitated substance.

Further substances that can be crystallized/precipitated in the process and apparatus of the present invention include, but are not limited to biopharmaceuticals as defined above, poorly water soluble drug compounds, such as, for example class 2 or class 4 pharmaceuticals. The present invention provides the ability to create drug crystals that are finer than typically produced by bulk crystallization (about 50 micron) or by bulk crystallization followed by various commonly used pharmaceutical milling processes (commonly about 10 micron) and thus the inventive process will enable poorly water soluble drugs to have a higher dissolution rate without the need/cost/contamination associated with milling processes or without the need to introduce solubility enhancing agents such as cyclodextrins or surfactants.

The pharmaceutical or biopharmaceutical substances may be those delivered via a pulmonary delivery mechanism, a parenteral delivery mechanism, a transdermal delivery mechanism, an oral delivery mechanism, an ocular delivery mechanism, a suppository or vaginal delivery mechanism, an aural delivery mechanism, a nasal delivery mechanism and an implanted delivery mechanism.

Further substance include metal particles, such as for example silver, gold, platinum, copper, tin, iron, lead, magnesium, titanium, mitures thereof and the like.

In addition, the present invention may be utilized for the production of any variety of small, high surface area particles that can be used as carrier particles for liquids or as seeds for crystallization or precipitation. The crystals/precipitate formed by the process of the invention can, in many cases, also be concurrently or subsequently coated with moisture barriers, taste-masking agents, or other additives that enhance the attributes of the crystallized pharmaceuticals. Likewise, the active substance crystals/particles can be formulated with other agents (such as excipients, surfactants, polymers) to provide the substance in an appropriate dosage form (e.g. tablets, capsules, etc) Thus, in the process of the present invention, in addition to the substance, a surfactant, emulsifier, stabilizer may be introduced as a third stream into the high shear zone, resulting in the stabilization of the precipitated dispersion.

In solvent/antisolvent methodology, the choice of particular solvent and antisolvent (or reactant/precipitant/cooling liquid or solution) can be made readily by a person skilled in the art considering the solubility characteristics of the compound to be precipitated. For example, an antisolvent can be, a water-soluble substance which is dissolved, for example, in water, and is precipitated by using a suitable water miscible antisolvent (e.g. acetone, isopropanol, dimethyl sulfoxide, etc., or mixtures thereof), for example, 20 weight % methanol with 80 weight %, ethanol. An additional antisolvent example could include, a less water-soluble substance which may be dissolved, for example, in an organic solvent such as light petroleum or ethyl acetate, and precipitated with, for example, with diethyl ether or cyclohexane.

A reactive precipitation/crystallization example could include a substance dissolved in water at high pH and precipitated with acidified water at a lower pH. An additional reactive example could include a rapid reaction between two inorganic ions, initially dissolved in separated aqueous solutions. An example of such a reactive precipitation or crystallization could take many forms, such as, the formation of a mineral salt (e.g. Al(OH)₃ or Ca₅(PO₄)₃OH, or a photonic material, such as CaF₂) or the crystallization/precipitation of a compound that forms a solid phase upon subjection to a pH change (e.g. adjusting the pH of a protein solution with an acid or base towards the isoelectric point of the protein, resulting in precipitation; additionally an example could be a carboxylic acid containing compound such as ibuprofen, which is poorly water soluble at low pH but considerably more soluble at higher pH).

A salting out precipitation/crystallization example could include a substance such as a protein or peptide dissolved in a buffered aqueous solution and precipitated or crystallized through mixing intimately with a solution of a salt dissolved for example in water (such as sodium chloride or ammonium sulphate).

A cooling driven crystallization/precipitation example could include a substance dissolved in a solvent and crystallized/precipitated by shock cooling, where the second liquid stream could be a refrigerated solvent such as for example water, ethylene glycol or ammonia.

Temperature of operation is one parameter that can affect solubility of substances, and thus, the yield of the process. For many materials, the yield can be maximized by operating at low temperatures. However, careful choice of antisolvents enables increased yields at room temperature operation of the process. Maximizing the yield of this process, however, is not an essential aspect of the process according to the present invention. The process of the present invention simply requires the temperature to be appropriate so that crystallization results. The temperature at which crystallization results is determined from solubility data, in some instances, solubility data is available in tables found, for example, in the Handbook of Chemistry and Physics, 73^{rd} edition, CRC Press or in scientific literature.

The rate of addition of the solvent(s) and anti-solvent(s) through the at least two inlet pipes may be controlled by any known method, a non-limiting example being a pump. The pump may be peristaltic in nature. Generally, those persons skilled in the art will recognize and understand those methods with which flow rates to typical rotor-stator devices may be restricted, such as including, but not limited to, using metering valves. Thus, those same methods are applicable to the present invention. The rates of solvent and antisolvent addition are limited only by the equipment used to control it. The fluids are added at a rate equivalent to the outflow, i.e., the sum of the inlet flow rates for the solvent(s) and antisolvent(s) is equal to the rate of the slurry exiting the process. Therefore, the system is generally considered continuous and "steady state" with respect to flows. The ratio of the two or more inlet streams may be any value as determined by the phase diagrams of the materials, as would be well known to one skilled in the art. The rate of crystal/precipitate formation generally depends on the degree of mixing. If one or more of the fluids is a slurry/suspension, seeding of the crystals/precipitates may result, wherein the crystals/precipitates formed according to the process are caused to crystallize/precipitate onto either the same substance being crystallized/precipitated, or onto a different substance that is, for example, suspended in at least one of the fluid streams being fed into the rotor-stator mixer.

Upon exiting the apparatus of the present invention, the precipitate/crystallized particles may be removed from the fluid mixture. Optionally, the precipitated compound may be dried by conventional methods generally known to persons skilled in the art. Examples of such methods include, but not limited to, spray-drying, oven-drying, flash-drying and air-drying. Optionally, prior to the drying step, the crystallized or precipitated particles may be separated out of the combined fluid mixture by using solid/liquid separation techniques generally known to persons skilled in the art, for example, filtration, settling, centrifugation, and the like. While the majority of crystals are removed from the system using the disclosed process, any substances that build up on the inner walls of the apparatus or its components may be isolated and/or discarded during routine maintenance.

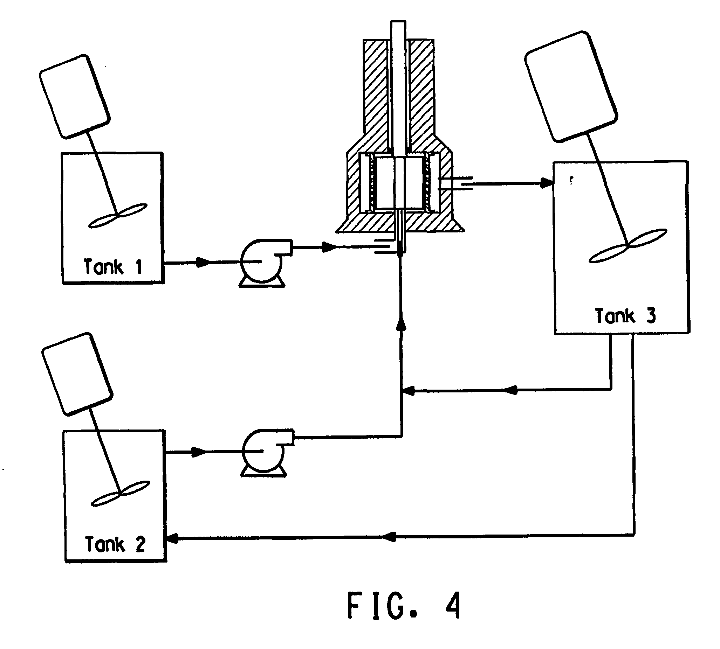

A recirculating configuration is also contemplated by the present invention, wherein the flow of crystals/precipitate from the outlet orifice may be circulated back into the apparatus of the present invention. As shown in Figure 4, Tank 1 and Tank 2 contain the feed stock, while product is collected in tank 3. A line is connected from tank 3 to one of the feed tanks or directly to the RS inlet. The recirculation configuration of a fraction of the product slurry may be of use in processes where seed crystals/precipitates are required to enable more rapid nucleation or to provide a surface for the growth of particles. Additionally, recirculation may be useful in situation where the product crystals/precipitates tend to flocculate and it is desired to keep them dispersed. Finally, there may be situations where the particles grow after exiting the apparatus of the invention and the recirculation enable the size of these growing crystals/precipitates to be maintained as small, through breakage.

An additional advantage of this apparatus is its ease of cleaning. A cleaning solution can be selected that will dissolve any internal encrustation and the shear force characteristic of the operating rotor-stator enables the device to self clean without need to disassemble and scrub internal surfaces.

As was previously discussed, and as will be evident to a person of ordinary skill in the art, the size of the crystals obtained according to the process of the present invention may be controlled by adjusting the parameters of the process. For example, increasing the rpm of the rotor-stator will often lead to finer particles, and adjusting the rate of addition and/or agitation will alter the particle size by altering the degree of supersaturation and mixing. Any one, several, or all of the process parameters may be adjusted in order to obtain the desired particle habit and/or size. A person of ordinary skill in the art may determine, using routine experimentation, the process parameters that are the most optimal in each individual situation.

Various methods may be employed in order to monitor the crystallinity of the particles of the present invention. Methods well known to persons skilled in the art include X-ray diffraction, differential scanning calorimetry (DSC) and scanning electron microscopy (SEM).

### EXAMPLES

The present invention is further defined in the following Examples, in which all parts and percentages are by weight. It should be understood that these Examples, while indicating preferred embodiments of the invention, are given by way of illustration only. From the above discussion and these Examples, one skilled in the art can ascertain the essential characteristics of this invention, and without departing from the spirit and scope thereof, can make various changes and modifications of the invention to adapt it to various usage and conditions.

Unless otherwise stated, all chemicals and reagents were used as received from Aldrich Chemical Company, Milwaukee, WI.

All references cited in the present disclosure are hereby specifically incorporated by reference in their entirety.

### Example 1 - Glycine

Glycine was dissolved in water to prepare 1 L of a 5% (w/w) aqueous solution. The solution was kept at room temperature +/-10 degrees C. This solution was fed to a Silverson Model L4RT-A Rotor-Stator in-line mixing assembly (Silverson Machines, Inc., East Longmeadow, MA, USA) at a flow rate of 190 mL/min. Simultaneously, anhydrous ethanol (>99%) was co-fed to this rotor-stator, also at 190 mUmin. The rotor-stator was operated at 10,000 rpm. The exit stream of the rotor-stator contained mother liquor and crystals of glycine with an elongated block-like habit, which were observed under a videomicroscope at magnifications up to 1000 X. The mean size of these crystals was measured to be 25 µm. A quenching solution of 50% water, 50% ethanol (saturated with dissolved glycine) was used to dissipate residual supersaturation of the exit stream from the rotor-stator.

### Example 2 - Glycine

The same procedure as in Example 1 was used, except the quenching solution contained 100% ethanol (saturated with glycine). Formed were crystals of sizes ranging from 25 µm to 60 µm and having an interpenetrant (or cruciform) twin habit.

### Example 3 - Glycine

The same procedure as in Example 1 was used, except where the rotor-stator speed was 5,000 rpm. Formed were block-like crystals where the mean size was 40 µm.

### Example 4 - Glycine

The same procedure as in Example 1 was used, except where the aqueous glycine solution was prepared to be 15% (w/w) in concentration. Formed were elongated block-like crystals where the mean size was 40 µm.

### Example 5 - Glycine

The same procedure as in Example 1 was used, except where the aqueous glycine solution was prepared to be 15% (w/w) in concentration and the rotor-stator speed was 5,000 rpm. Formed were elongated block-like crystals where the mean size was 70 µm.

### Example 6 - Glycine

The same procedure as in Example 1 was used, except where the aqueous glycine solution was prepared to be 15% (w/w) in concentration and the rotor-stator speed was 0 rpm. Formed were needle like crystals where the mean length was 300 µm.

### Example 7 - Glycine

The same procedure as in Example 1 was used, except where the aqueous glycine solution was prepared to be 15% (w/w) in concentration and the flow rate of aqueous glycine to the rotor-stator was 21 mL/min and the flow rate of anhydrous ethanol to the rotor-stator was 190 mL/min. Produced were fine, rounded crystals of a narrow size distribution, where the mean size was 6 µm.

### Example 8 - Glycine

The same procedure as in Example 7 was used, except the feed rate of the 15 % (w/w) aqueous glycine solution was 14 mL/min and the feed rate of the anhydrous ethanol antisolvent was 190 mUmin. A quenching solution of anhydrous ethanol (>99%) was used. Produced were fine, rounded crystals of a narrow size distribution, where the mean size of primary crystals was found to be 4.4 µm, as determined by image analysis.

### Example 9 - aspirin

Salicylic acid (aspirin) was dissolved in anhydrous ethanol (>99%) at a concentration of 24.8 % (w/w). This solution was fed to the same apparatus as configured in Example 1. Water was co-fed as the antisolvent. Preliminary testing without use of a quenching solution noted a sensitivity of aspririn crystal growth kinetics and crystallizable mass that in some circumstance led to crystals continuing to grow up to 78.6 µm after exiting the high shear zone. Conditions were determined where this effect was minimized without use of a quenching solution. For instance, the apparatus was operated at 10,000 rpm, with a feed rate of ethanolic aspirin solution at 133 mL/min and a co-feed rate of water as an antisolvent at 9 mL/min. The product crystals were found to have a mean size of 3.3 µm determined by Malvern Mastersizer 2000 (version 2.00) and a primary particle size determined by image analysis of 2.7 µm.

### Example 10 - Silver

Silver particles were prepared using the apparatus as described in Example 1. Two solutions were fed: a silver containing solution and a reducing solution. For experiments 10A through 10E, the silver containing solution was comprised of 105 g silver nitrate, 88 ml monoethanolamine, and 1 liter water. The reducing solution was comprised of 17 g hydroquinone, 300 ml monoethanolamine, and 1 liter water. For experiment 10F, the above solutions were diluted ten fold (silver containing solution: 10.5 g silver nitrate and 8.8 ml monoethanolamine and 1 liter water; reducing solution: 1.7 g hydroquinone, and 30 ml monoethanolamine in 1 liter water). For experiment 10G, the solutions were diluted 100 fold (silver containing solution: 1.05 g silver nitrate and 0.88 ml monoethanolamine and 1 liter water; reducing solution: 0.17 g hydroquinone and 3 ml monoethanolamine in 1 liter water).

The silver containing and reducing solutions were co-fed to the apparatus at equal flow rates. The table below gives the flow rates, speed of the rotor-stator mixer and the mean particle size as determined by Malvern Mastersizer 2000 (version 2.00). The size for the product of experiment 10G is reported as the size of the primary mode. Larger particles were present in the distribution, but they are believed to be aggregates of the 0.4 µm primary particles.

| Experiment | Solution flow rate (mL/min) | Rotor speed (rpm) | Mean particle size (µm) |
|---|---|---|---|
| 10A | 20 | 5,000 | 2.4 |
| 10B | 20 | 7500 | 2.3 |
| 10C | 20 | 9500 | 2.2 |
| 10D | 50 | 9500 | 3.4 |
| 10E | 150 | 9500 | 4.5 |
| 10F | 50 | 9500 | 1.7 |
| 10G | 50 | 9500 | Primary mode size: 0.4 µm |

### Example 11 - Soy Protein

Soluble soy proteins were extracted from 193.8 g of defatted white soy flake (supplied by DuPont Protein Technologies, St. Louis Missouri) with 1500 g of deionized water at pH 6.6. After gentle agitation for 20 minutes, the slurry was centrifuged for 10 minutes at 9,000 rpm in a Sorval RC26Plus centrifuge, with GS-3 rotor. The supernatant was light brown in color and substantially free of particles or visible dispersions. The supernatant containing soluble soy proteins was fed to the same Rotor-Stator mixer apparatus of Example 1. The solution was kept at room temperature +/- 10 degrees C. This solution was fed to the apparatus of Example 1 at a flow rate of 115 mL/min. Simultaneously, dilute hydrochloric acid (0.015M) was co-fed to this rotor-stator, at 115 mL/min. The rotor-stator was operated at 11,000 rpm. The exit stream of the rotor-stator contained a slurry of precipitated soy protein particles at pH 5.5. The soy protein particles were observed under a microscope and noticed to have a very small size, typically spherical or globular. Over time the primary soy protein particles would tend to flocculate. Hence their primary particle size was determined by light scattering in a Malvern Mastersizer 2000 (version 2.00) with sonication to disperse the flocs during size analysis. The volume mean particle size was 2.6 µm, however the size distribution was notably bimodal. The smallest mode indicating the mean primary particle size and the second mode indicating the mean floc size during size analysis. Thus the mean primary soy protein particle size was determined to be 1.5 µm and the mean floc size was determined to be 4.0 µm.

### Example 12 - Soy Protein

The same procedure and materials as in Example 11 was followed, except the feed rate of soy protein extract was 115 mL/min and the 0.015M hydrochloric acid solution was co-fed at a rate of 87 mL/min. The exit stream of the rotor-stator contained a slurry of precipitated soy protein particles at pH 5.6. The speed of the rotor-stator mixer was 500 rpm. The particle size was measured by the same method as example 11. The volume mean particle size was 0.84 µm; the primary particles were determined to have a mean size of 0.2 µm and the mean floc size was determined to be 1.5 µm.

## Claims

1. A crystallization/precipitation apparatus (1) comprising:
a housing (2) having at least a first cavity (3); a stator (5) having a plurality of apertures (13), an interior wall portion (23), and an outer wall portion (24), wherein the stator resides within the first cavity; a rotor (6), wherein the rotor is connected to a rotatably mounted drive shaft (8) and is contained within a rotor-swept volume (22); at least two inlet pipes (9, 10) wherein the at least two inlet pipes introduce at least two fluid streams into the rotor-swept volume; at least one entry port (12); and an outlet orifice (11); said apparatus **characterised in that** the rotor further comprises at least one rotor blade (7) that extends radially away from the rotatably mounted drive shaft and at least one rotor blade tip (18) wherein the at least one rotor blade tip is separated from the interior wall portion of the stator by a shear gap (17).

2. The apparatus of claim 1, wherein the stator is cylindrical.

3. The apparatus of claim 1, wherein the shear gap existing between at least one blade tip and an interior wall portion of the stator ranges from 0.01 mm to 10 mm.

4. The apparatus of claim 3, wherein the shear gap existing between at least one blade tip and the interior wall portion of the stator is 1 mm.

5. The apparatus of claim 1, wherein the rotatably mounted drive shaft is hollow.

6. The apparatus of claim 1, wherein the rotor is hollow.

7. The apparatus of claim 1, wherein the at least one rotor blade is hollow.

8. The apparatus of claim 2, wherein the cylindrical stator has apertures or teeth.

9. The apparatus of claim 2, wherein the cylindrical stator has surface modifications.

10. The apparatus of claim 1, said housing being a stainless steel housing having the first cavity and a second cavity;
said stator being a stainless steel cylindrical stator;
said rotor being a stainless steel rotor;
said blade tip being separated from the interior wall portion of the stator by a shear gap of 1 mm; and
the at least two inlet pipes being at least two multi-axial inlet pipes; and wherein the rotatably mounted drive shaft extends through the second cavity.

11. A process for crystallizing/precipitating particles comprising the steps of: feeding at least two fluids into the apparatus of claim 1, wherein at least one first fluid is a solvent comprising at least one dissolved substance that is to be crystallized/precipitated into particles and at least one second fluid comprising an anti-solvent, said solvent and anti-solvent being miscible; mixing said first and second fluids using a shear force in a high shear zone wherein the at least one dissolved substance is caused to crystallize/precipitate into particles from said first solution on being mixed with said second fluid in the high shear zone; and causing the mixed first and second fluids and the particles to exit the apparatus of claim 1.

12. The process of claim 11 wherein the particles have a particle size ranging from 100 nm to 100 µm.

13. The process of claim 12, wherein the particles have a particle size ranging from 100 nm to 10 µm.

14. The process of claim 13, wherein the particles have a particle size ranging from 10 nm to 10 µm.

15. The process of claim 11, wherein the nominal shear rate is up to about 1,000,000 reciprocal seconds.

16. The process of claim 11, wherein the substance is a food or food ingredient

17. The process of claim 11, wherein the substance is selected from the group consisting of carbohydrates, polysaccharides, oligosaccharides, disaccharides, monosaccharides, proteins, peptides, amino acids, lipids, vitamins, minerals, salts, food colors, enzymes, sweeteners, anti-caking agents, thickeners, emulsifiers, stabilizers, antimicrobial agents, antioxidants and mixtures thereof

18. The process of claim 11 wherein the substance is a metal particle.

19. The process of claim 11, wherein the substance is a photonic material.

20. The process of claim 11, wherein the substance is a pharmaceutical or biopharmaceutical.

21. The process of claim 20, wherein the substance is a poorly water soluble drug compound.

22. The process of claim 14, wherein the particles are a pharmaceutical or biopharmaceutical compound.

23. The process of claim 18, wherein the metal particle is selected from the group consisting of silver, gold, platinum, copper, tin, iron, lead, magnesium, titanium and mixtures thereof.

24. A process for crystallizing/precipitating a soy protein comprising the steps of:
feeding a first solvent fluid comprising deionized water having soy proteins dissolved therein and a second fluid comprising dilute acid into the apparatus of claim 1, wherein the first solvent fluid is a solvent comprising soy proteins that are to be precipitated into particles and the second fluid comprises an anti-solvent, said solvent and anti-solvent being miscible; mixing said first and second fluids in a high shear zone wherein the soy proteins are caused to crystallize/precipitate into particles from said first solution upon being mixed with said second fluid in the high shear zone; and causing the mixed first and second fluids and the soy protein particles to exit the apparatus of claim 1.

25. A process for crystallizing/precipitating particles comprising the steps of: feeding at least two fluids into a rotor-stator apparatus of claim 1, wherein at least one first fluid is a solvent comprising at least one dissolved substance that is to be precipitated into particles and at least one second fluid comprising an anti-solvent, said solvent and anti-solvent being miscible; mixing said first and second fluids using a shear force in a high shear zone wherein the at least one dissolved substance is caused to crystallize/precipitate into particles from said first solution on being mixed with said second fluid in the high shear zone; and causing the mixed first and second fluids and the particles to exit the rotor-stator apparatus of claim 1.

26. The process of claim 25, wherein the particles have a particle size ranging from 100 nm to 100 µm.

27. The process of claim 25, wherein the substance is a food or food ingredient.

28. The process of claim 25, wherein the substance is selected from the group consisting of carbohydrates, polysaccharides, oligosaccharides, disaccharides, monosaccharides, proteins, peptides, amino acids, lipids, vitamins, minerals, salts, food colors, enzymes, sweeteners, anti-caking agents, thickeners, emulsifiers, stabilizers, antimicrobial agents, antioxidants and mixtures thereof

29. The process of claim 25, wherein the substance is a metal particle.

30. The process of claim 29, wherein the metal particle is selected from the group consisting of silver, gold, platinum, copper, tin, iron, lead, magnesium, titanium and mixtures thereof.

31. The process of claim 25, wherein the substance is a photonic material.

32. The process of claim 25, wherein the substance is a pharmaceutical or biopharmaceutical.

33. The process of claim 32, wherein the substance is a poorly water soluble drug compound.

## Revendications

1. Dispositif de cristallisation/précipitation (1) comprenant:
un logement (2), ayant au moins une première cavité (3); un stator (5) ayant une pluralité d'ouvertures (13), une portion de paroi intérieure (23) et une portion de paroi externe (24), où le stator réside au sein de la première cavité; un rotor (6) où le rotor est connecté à un arbre d'entraînement (8), monté de manière à pouvoir tourner, et est contenu au sein du volume balayé par le rotor (22); au moins deux conduits d'entrée (9, 10), où les au moins deux conduits introduisent au moins deux courants de fluide dans le volume balayé par le rotor; au moins un port d'entrée (12); et un orifice de sortie (11); ledit dispositif étant **caractérisé en ce que** le rotor comprend en outre au moins une pale de rotor (7) qui se prolonge de manière radiale à l'écart de l'arbre d'entraînement monté de manière à pouvoir tourner et au moins un bout de pale de rotor (18) où le au moins un bout de pale de rotor est séparé de la portion de paroi intérieure du stator par un entrefer de cisaillement (17).

2. Dispositif selon la revendication 1, dans lequel le stator est cylindrique.

3. Dispositif selon la revendication 1, dans lequel l'entrefer de cisaillement existant entre au moins un bout de pale et une portion de paroi intérieure du stator va de 0,01 mm à 10 mm.

4. Dispositif selon la revendication 3, dans lequel l'entrefer de cisaillement existant entre au moins un bout de pale et une portion de paroi intérieure du stator est de 1 mm.

5. Dispositif selon la revendication 1, dans lequel l'arbre d'entraînement monté de manière à pouvoir tourner est creux.

6. Dispositif selon la revendication 1, dans lequel le rotor est creux.

7. Dispositif selon la revendication 1, dans lequel la au moins une pale de rotor est creuse.

8. Dispositif selon la revendication 2, dans lequel le stator cylindrique a des ouvertures ou des dents.

9. Dispositif selon la revendication 2, dans lequel le stator cylindrique a des modifications de surface.

10. Dispositif selon la revendication 1, ledit logement étant un logement en acier inoxydable ayant la première cavité et le deuxième cavité;
ledit stator étant un stator cylindrique en acier inoxydable;
ledit rotor étant un rotor en acier inoxydable;
ledit bout de pale étant séparé de la portion de paroi intérieure du stator par un entrefer de cisaillement de 1 mm; et
les au moins deux conduits étant au moins deux conduits d'admission multi-axiaux; et dans lequel l'arbre d'entraînement monté de manière à pouvoir tourner se prolonge à travers la deuxième cavité.

11. Procédé de cristallisation/de précipitation de particules comprenant les étapes: d'alimentation d'au moins deux fluides dans l'dispositif selon la revendication 1, dans lequel au moins un premier fluide est un solvant comprenant au moins une substance dissoute qui est à cristalliser/à précipiter sous forme de particules et au moins un deuxième fluide comprenant un anti-solvant, lesdits solvant et anti-solvant étant miscibles; de mélange desdits premier et deuxièmes fluides par utilisation d'une force de cisaillement dans une zone à cisaillement élevé où la au moins une substance dissoute est forcée de se cristalliser/de se précipiter sous forme de particules à partir de ladite première solution au moment du mélange avec ledit deuxième fluide dans la zone à cisaillement élevé; et forcer les premier et deuxième fluides mélangés et les particules de sortir de l'dispositif selon la revendication 1.

12. Procédé selon la revendication 1, dans lequel les particules ont une taille de particule allant de 100 nm à 100 µm.

13. Procédé selon la revendication 2, dans lequel les particules ont une taille de particule allant de 100 nm à 10 µm.

14. Procédé selon la revendication 3, dans lequel les particules ont une taille de particule allant de 10 nm à 10 µm.

15. Procédé selon la revendication 1, dans lequel la vitesse de cisaillement nominale va jusqu'à environ 1 000 000 secondes réciproques.

16. Procédé selon la revendication 1, dans lequel la substance est un aliment ou un constituant d'aliment.

17. Procédé selon la revendication 1, dans lequel la substance est sélectionnée du groupe constitué des hydrates de carbone, des polysaccharides, des oligosaccharides, des disaccharides, des monosaccharides, des protéines, des peptides, des aminoacides, des lipides, des vitamines, des minéraux, des sels, des colorants alimentaires, des enzymes, des édulcorants, des agents anti-agglutinants, des épaississants, des émulsifiants, des agents stabilisants, des agents anti-microbiens, des agents anti-oxydants et des mélanges de ces derniers.

18. Procédé selon la revendication 1, dans lequel la substance est une particule de métal.

19. Procédé selon la revendication 1, dans lequel la substance est un matériau photonique.

20. Procédé selon la revendication 1, dans lequel la substance est un composé pharmaceutique ou bio-pharmaceutique.

21. Procédé selon la revendication 20, dans lequel la substance est un composé de médicament médiocrement hydrosoluble.

22. Procédé selon la revendication 14, dans lequel les particules sont un composé pharmaceutique ou bio-pharmaceutique.

23. Procédé selon la revendication 18, dans lequel la particule de métal est sélectionné parmi le groupe constitué d'argent, d'or, de platine, de cuivre, d'étain, de fer, de plomb, de magnésium, de titane et de mélanges de ces derniers.

24. Procédé de cristallisation/de précipitation d'une protéine de soja comprenant les étapes: d'alimentation d'un premier fluide de solvant comprenant l'eau désionisée ayant des protéines de soja dissoutes dans cette dernière et un deuxième fluide comprenant l'acide dilué dans le dispositif selon la revendication 1, dans lequel le premier solvant est un solvant comprenant des protéines de soja qui sont à précipiter sous forme de particules et le deuxième fluide comprend un anti-solvant, lesdits solvant et anti-solvant étant miscibles; de mélange desdits premier et deuxième fluides dans une zone à cisaillement élevé où les protéines de soja sont forcées de se cristalliser/ de se précipiter sous forme de particules à partir de ladite première solution après mélange avec ledit deuxième fluide dans la zone à cisaillement élevé, et l'étape consistant à forcer les premier et deuxième fluides et les particules de protéines de soja à sortir du dispositif selon la revendication 1.

25. Procédé de cristallisation/de précipitation de particules comprenant les étapes: d'alimentation d'au moins deux fluides dans un dispositif rotor-stator selon la revendication 1, dans lequel au moins un premier fluide est un solvant comprenant au moins une substance dissoute qui est à précipiter sous forme de particules et au moins un deuxième fluide comprenant un anti-solvant, lesdits solvant et anti-solvant étant miscibles; de mélange desdits premier et deuxième fluides par utilisation d'une force de cisaillement dans une zone à cisaillement élevé dans laquelle la au moins une substance dissoute est forcée de se cristalliser/de se précipiter sous forme de particules à partir de ladite première solution à la suite du mélange avec ledit deuxième fluide dans la zone à cisaillement élevé; et forcer les premier et deuxième fluides et les particules à sortir du dispositif rotor-stator selon la revendication 1.

26. Procédé selon la revendication 25, dans lequel les particules ont une taille de particule allant de 100 nm à 100 µm.

27. Procédé selon la revendication 25, dans lequel la substance est un aliment ou un constituant alimentaire.

28. Procédé selon la revendication 25, dans lequel la substance est sélectionnée du groupe constitué des hydrates de carbone, des polysaccharides, des oligosaccharides, des disaccharides, des monosaccharides, des protéines, des peptides, des aminoacides, des lipides, des vitamines, des minéraux, des sels, des colorants alimentaires, des enzymes, des édulcorants, des agents anti-agglutinants, des épaississants, des émulsifiants, des agents stabilisants, des agents anti-microbiens, des agents anti-oxydants et des mélanges de ces derniers.

29. Procédé selon la revendication 25, dans lequel la substance est une particule de métal.

30. Procédé selon la revendication 29, dans lequel la particule de métal est sélectionné parmi le groupe constitué d'argent, d'or, de platine, de cuivre, d'étain, de fer, de plomb, de magnésium, de titane et de mélanges de ces derniers.

31. Procédé selon la revendication 25, dans lequel la substance est un matériau photonique.

32. Procédé selon la revendication 25, dans lequel la substance est un composé pharmaceutique ou bi-pharmaceutique.

33. Procédé selon la revendication 32, dans lequel la substance est un composé de médicament médiocrement hydrosoluble.

## Patentansprüche

1. Kristallisations-/Abscheidungs vorrichtung (1), die aufweist:
ein Gehäuse (2) mit mindestens einem ersten Hohlraum (3), einen Stator (5) mit mehreren Öffnungen (13), einem Innenwandabschnitt (23) und einem Außenwandabschnitt (24), wobei der Stator innerhalb des ersten Hohlraums (3) angeordnet ist; einen Rotor (6),
wobei der Rotor mit einer drehbar montierten Antriebswelle (8) verbunden und in einem Rotordurchlaufvolumen (22) enthalten ist; mindestens zwei Einlassrohre (9, 10), wobei die mindestens zwei Einlassrohre mindestens zwei Fluidströme in das Rotordurchlaufvolumen einleiten; mindestens eine Eintrittsöffnung (12); und eine Auslassöffnung (11); wobei die Vorrichtung **dadurch gekennzeichnet ist, daß** der Rotor ferner mindestens ein radial von der drehbar montierten Antriebswelle ausgehendes Rotorblatt (7) und mindestens eine Rotorblattspitze (18) aufweist, wobei die mindestens eine Rotorblattspitze von dem Innenwandabschnitt des Stators durch einen Scherungszwischenraum (17) getrennt ist.

2. Vorrichtung nach Anspruch 1, wobei der Stator zylinderförmig ist.

3. Vorrichtung nach Anspruch 1, wobei der zwischen mindestens einer Blattspitze und einem Innenwandabschnitt vorhandene Scherungszwischenraum im Bereich von 0,01 mm bis 10 mm liegt.

4. Vorrichtung nach Anspruch 3, wobei der zwischen mindestens einer Blattspitze und dem Innenwandabschnitt des Stators vorhandene Scherungszwischenraum 1 mm beträgt.

5. Vorrichtung nach Anspruch 1, wobei die drehbar montierte Antriebswelle hohl ist.

6. Vorrichtung nach Anspruch 1, wobei der Rotor hohl ist.

7. Vorrichtung nach Anspruch 1, wobei das mindestens eine Rotorblatt hohl ist.

8. Vorrichtung nach Anspruch 2, wobei der zylinderförmige Stator Öffnungen oder Zähne aufweist.

9. Vorrichtung nach Anspruch 2, wobei der zylinderförmige Stator Oberflächenmodifikationen aufweist.

10. Vorrichtung nach Anspruch 1, wobei das Gehäuse ein Edelstahlgehäuse mit einem ersten Hohlraum und einem zweiten Hohlraum ist;
wobei der Stator ein zylinderförmiger Edelstahlstator ist;
wobei der Rotor ein Edelstahlrotor ist;
wobei die Blattspitze durch einen Scherungszwischenraum von 1 mm vom Innenwandabschnitt des Stators getrennt ist; und
wobei die mindestens zwei Einlassrohre mindestens zwei mehrachsige Einlassrohre sind; und wobei sich die drehbar montierte Antriebswelle durch den zweiten Hohlraum erstreckt.

11. Verfahren zur Kristallisation/Ausfällung von Teilchen mit den folgenden Schritten: Einspeisen von mindestens zwei Fluiden in die Vorrichtung gemäß Anspruch 1, wobei mindestens ein erstes Fluid ein Lösungsmittel ist, das mindestens eine gelöste Substanz aufweist, die zu Teilchen kristallisiert/ausgefällt werden soll, und wobei mindestens ein zweites Fluid ein Antilösungsmittel bzw. Fällungsmittel aufweist, wobei das Lösungsmittel und das Antilösungsmittel mischbar sind; Vermischen der ersten und zweiten Fluide unter Anwendung einer Scherkraft in einer Zone hoher Scherung, wobei die mindestens eine gelöste Substanz aus der ersten Lösung beim Vermischen mit dem zweiten Fluid in der Zone hoher Scherung zu Teilchen kristallisiert/ausgefällt wird, und Veranlassen, daß die vermischten ersten und zweiten Fluide und die Teilchen aus der Vorrichtung gemäß Anspruch 1 austreten.

12. Verfahren nach Anspruch 11, wobei die Teilchen eine Teilchengröße im Bereich von 100 nm bis 100 µm aufweisen.

13. Verfahren nach Anspruch 12, wobei die Teilchen eine Teilchengröße im Bereich von 100 nm bis 10 µm aufweisen.

14. Verfahren nach Anspruch 13, wobei die Teilchen eine Teilchengröße im Bereich von 10 nm bis 10 µm aufweisen.

15. Verfahren nach Anspruch 11, wobei die Nennscherrate bis zu etwa 1 000 000 s ⁻¹ beträgt.

16. Verfahren nach Anspruch 11, wobei die Substanz ein Nahrungsmittel oder Nahrungsmittelzusatzstoff ist.

17. Verfahren nach Anspruch 11, wobei die Substanz aus der Gruppe ausgewählt ist, die aus Kohlehydraten, Polysacchariden, Oligosacchariden, Disacchariden, Monosacchariden, Proteinen, Peptiden, Aminosäuren, Lipiden, Vitaminen, Mineralstoffen, Salzen, Lebensmittelfarbstoffen, Enzymen, Süßungsmitteln, Antibackmitteln, Verdickungsmitteln, Emulgatoren, Stabilisatoren, antimikrobiellen Wirkstoffen, Antioxidantien und deren Gemischen besteht.

18. Verfahren nach Anspruch 11, wobei die Substanz ein Metallteilchen ist.

19. Verfahren nach Anspruch 11, wobei die Substanz ein photonisches Material ist

20. Verfahren nach Anspruch 11, wobei die Substanz ein Pharmazeutikum oder Biopharmazeutikum ist.

21. Verfahren nach Anspruch 20, wobei die Substanz eine schlecht wasserlösliche Arzneimittelverbindung ist.

22. Verfahren nach Anspruch 14, wobei die Teilchen eine pharmazeutische oder biopharmazeutische Verbindung sind.

23. Verfahren nach Anspruch 18, wobei das Metallteilchen aus der Gruppe ausgewählt ist, die aus Silber, Gold, Platin, Kupfer, Zinn, Eisen, Blei, Magnesium, Titan und Gemischen daraus besteht.

24. Verfahren zur Kristallisation/Ausfällung eines Sojaproteins mit den folgenden Schritten: Einspeisen eines ersten Lösungsmittelfluids, das entionisiertes Wasser mit darin gelösten Sojaproteinen aufweist, und eines zweiten Fluids mit verdünnter Säure in die Vorrichtung gemäß Anspruch 1, wobei das erste Lösungsmittelfluid ein Lösungsmittel ist, das Sojaproteine aufweist, die zu Teilchen ausgefällt werden sollen, und wobei das zweite Fluid ein Antilösungsmittel aufweist, wobei das Lösungsmittel und das Antilösungsmittel mischbar sind; Vermischen der ersten und zweiten Fluide in einer Zone hoher Scherung, wobei die Sojaproteine aus der ersten Lösung beim Vermischen mit dem zweiten Fluid in der Zone hoher Scherung zu Teilchen kristallisiert/ausgefällt werden; und Veranlassen, daß die vermischten ersten und zweiten Fluide und die Sojaproteinteilchen aus der Vorrichtung gemäß Anspruch 1 austreten.

25. Verfahren zur Kristallisation/Ausfällung von Teilchen, mit den folgenden Schritten: Einspeisen von mindestens zwei Fluiden in eine Rotor-Stator-Vorrichtung gemäß Anspruch 1, wobei mindestens ein erstes Fluid ein Lösungsmittel mit mindestens einer darin gelösten Substanz ist, die zu Teilchen auszufällen ist, und mindestens ein zweites Fluid ein Antilösungsmittel aufweist, wobei das Lösungsmittel und das Antilösungsmittel mischbar sind; Vermischen der ersten und zweiten Fluide unter Anwendung einer Scherkraft in einer Zone hoher Scherung, wobei die mindestens eine gelöste Substanz aus der ersten Lösung beim Vermischen mit dem zweiten Fluid in der Zone hoher Scherung kristallisiert/ausgefällt wird; und Veranlassen, daß die ersten und zweiten Fluide und die Teilchen aus der Rotor-Stator-Vorrichtung gemäß Anspruch 1 austreten.

26. Verfahren nach Anspruch 25, wobei die Teilchen eine Teilchengröße im Bereich von 100 nm bis 100 µm aufweisen.

27. Verfahren nach Anspruch 25, wobei die Substanz ein Nahrungsmittel oder Nahrungsmittelzusatzstoff ist.

28. Verfahren nach Anspruch 25, wobei die Substanz aus der Gruppe ausgewählt ist, die aus Kohlehydraten, Polysacchariden, Oligosacchariden, Disacchariden, Monosacchariden, Proteinen, Peptiden, Aminosäuren, Lipiden, Vitaminen, Mineralstoffen, Salzen, Lebensmittelfarbstoffen, Enzymen, Süßungsmitteln, Antibackmitteln, Verdickungsmitteln, Emulgatoren, Stabilisatoren, antimikrobiellen Wirkstoffen, Antioxidantien und deren Gemischen besteht.

29. Verfahren nach Anspruch 25, wobei die Substanz ein Metallteilchen ist.

30. Verfahren nach Anspruch 29, wobei das Metallteilchen aus der Gruppe ausgewählt ist, die aus Silber, Gold, Platin, Kupfer, Zinn, Eisen, Blei, Magnesium, Titan und Gemischen daraus besteht.

31. Verfahren nach Anspruch 25, wobei die Substanz ein photonisches Material ist.

32. Verfahren nach Anspruch 25, wobei die Substanz ein Pharmazeutikum oder Biopharmazeutikum ist.

33. Verfahren nach Anspruch 32, wobei die Substanz eine schlecht wasserlösliche Arzneimittelverbindung ist.
